(19) Europäisches Patentamt
European Patent Office
Office européen des brevets

(11) **EP 3 156 086 B1**

(12) **EUROPÄISCHE PATENTSCHRIFT**

(45) Veröffentlichungstag und Bekanntmachung des Hinweises auf die Patenterteilung:
**03.03.2021 Patentblatt 2021/09**

(51) Int Cl.:
***A61M 1/16*** *(2006.01)*

(21) Anmeldenummer: **16192616.7**

(22) Anmeldetag: **06.10.2016**

(54) **KOMPENSATION VON UF-FEHLBILANZEN**

COMPENSATION OF UF BALANCING ERRORS

COMPENSATION DES ERREURS DE BILAN D'UF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2015 DE 102015117396**

(43) Veröffentlichungstag der Anmeldung:
**19.04.2017 Patentblatt 2017/16**

(73) Patentinhaber: **B. Braun Avitum AG**
**34212 Melsungen (DE)**

(72) Erfinder:
• **Meibaum, Jörn, Dr.**
**34253 Lohfelden (DE)**
• **Meierholz, Katharine**
**34212 Melsungen (DE)**
• **Wagner, Andre**
**34117 Kassel (DE)**

(74) Vertreter: **Winter, Brandl, Fürniss, Hübner, Röss, Kaiser, Polte - Partnerschaft mbB Patent- und Rechtsanwaltskanzlei**
**Alois-Steinecker-Straße 22**
**85354 Freising (DE)**

(56) Entgegenhaltungen:
EP-A2- 0 597 817          WO-A1-2011/038858
DE-A1- 2 838 414          DE-A1-102014 003 619
US-A1- 2005 000 868

**Beschreibung**

Die Erfindung betrifft ein nicht beanspruchtes Verfahren zur Bestimmung einer Fehlbilanz, insbesondere einer Ultrafiltrations-Fehlbilanz, einer Dialysemaschine mit einem Dialysator, wobei zum Dialysator fließende Frischdialysierflüssigkeit und vom Dialysator abströmende Brauchdialysierflüssigkeit mittels zumindest einer Bilanzierungskammer bilanziert wird oder werden. Sie betrifft des Weiteren eine Dialysemaschine mit einem Dialysator und zumindest einer Bilanzierungskammer zur Bilanzierung von zum Dialysator fließende Frischdialysierflüssigkeit und vom Dialysator abströmende Brauchdialysierflüssigkeit.

Bei heutigen Dialyseanwendungen werden Dialysemaschinen genutzt, die Bilanzierungseinrichtungen umfassen. Diese überwachen und stellen sicher, dass ein dem Dialysator über einen bestimmten Zeitraum zugeführtes Flüssigkeitsvolumen einem dem Dialysator über den gleichen Zeitraum entnommenem Flüssigkeitsvolumen entspricht. Bei einigen Anwendungen kann eine Dosierungspumpe oder Ultrafiltrationspumpe bewirken, dass dem Patienten über die Dauer einer Therapie ein definiertes Ultrafiltrationsvolumen abgenommen wird. Dieses wird bei der Bilanzierung nicht direkt erfasst bzw. selbst nicht bilanziert. Zusammenfassend kann man also sagen, dass mittels der Bilanzierungseinrichtung überwacht wird, dass dem Patienten keine Flüssigkeit oder nur genau eine beabsichtigte Flüssigkeitsmenge entzogen wird.

Aus dem Stand der Technik sind Bilanzierungsverfahren ohne Bilanzkammern mit Membranen bekannt, zum Beispiel mit Coriolis Messzellen oder Bilanzierungsverfahren mit hochgenauen, wechselseitigen Pumpen. Ein anderes bekanntes Bilanzierungssystem einer Dialysemaschine besteht aus zwei Bilanzierungskammern, die einem Dialysator abwechselnd Dialysierflüssigkeit zu- bzw. abführen. Jede Bilanzkammer besitzt eine Membran, die sich jeweils in entgegengesetzter Richtung ausdehnen. Bedingt beispielsweise durch das Material der Membranen, mögliche Luftansammlungen in den Kammern oder die Nachgiebigkeit bestimmter Baugruppen wie Schläuche, Kammern etc. kann das Bilanzkammersystem sehr sensibel auf Druckunterschiede reagieren. Nur wenn der am Bilanzkammereingang anliegende Druck zum am Bilanzkammerausgang anliegenden Druck identisch ist, funktioniert das Bilanzierungssystem ordnungsgemäß und fehlerfrei und ist die Bilanz korrekt. Zur Erzeugung geeigneter Druckverhältnisse werden Drosseln (Konstantdruckventile) eingesetzt, die bei einem definierten Fluss einen definierten Druck erzeugen. Auf diese Weise können zumindest für Standardeinstellungen konstante Druckverhältnisse garantiert werden. Es ist jedoch ein Nachteil von Konstantdruckventilen, dass diese ein relativ großes Toleranzfeld (in der Regel in einer Größenordnung von ca. +/- 50 mmHg) besitzen. Es kann daher insbesondere bei von Standardeinstellungen abweichenden Volumenströmen zu abweichenden Drücken vor und nach der Bilanzierungseinrichtung kommen, obwohl die genutzten Konstantdruckventile identische Einstellungen aufweisen. Derartige Verhältnisse führen zu einer Fehlbilanzierung.

Die US 2005/0000868 A1 offenbart ein Verfahren und eine Vorrichtung, zum Einstellen eines volumetrischen Strömungsausgleichssystems für ein extrakorporales Blutbehandlungssystem, die ein Drucksignal empfangen und einen Kompensationsfaktor berechnet, der verwendet wird, um die relativen Strömungsraten der volumetrisch ausgleichenden Fluide einzustellen.

Ein Problem bekannter Dialysemaschinen und Verfahren ist, dass ein dem Patienten entzogenes Ultrafiltrationsvolumen von der Bilanzierungseinrichtung nicht direkt erfasst wird. Das dem Patienten entzogene Flüssigkeitsvolumen sollte also allein von der Ultrafiltrationspumpe abhängen. In der Praxis kommt es jedoch vor allem bei Bilanzkammersystemen mit Membranen zu Fehlultrafiltration, was auf einer Fehlbilanzierung durch das System beruht, die wieder zum Beispiel durch variierenden Druck, variierende Temperatur und/oder variierende Volumenstromraten innerhalb des Bilanzierungssystems hervorgerufen wird. Die mittels einer Ultrafiltrationspumpe dem Dialysierflüssigkeitskreislauf abgezogene Ultrafiltrationsmenge wird mit Hilfe der Bilanzierung durch das Bilanzierungssystem quasi indirekt gesteuert oder geregelt. Insbesondere durch Änderung der Gegendrücke, die auf die Bilanzierungskammern wirken, kann es im Rahmen einer Dialysebehandlung mit Ultrafiltration und die so hervorgerufene Fehlbilanzierung bei bekannten System und Verfahren zu Abweichungen von Ultrafiltrationsmengen (UF Fehlmenge) kommen.

Die Ultrafiltration ist auch abhängig von den an der Bilanzkammer vorliegenden Temperaturverhältnissen, insbesondere von Temperaturdifferenzen zwischen Bilanzierungskammer-Eingang und Bilanzierungskammer-Ausgang. Dort oder an anderer Stelle vorliegende Temperaturänderungen oder -differenzen ziehen eine Änderung des Volumens der bilanzierten Flüssigkeit mit sich, die durch Änderung der Dichte der Flüssigkeit in Abhängigkeit der Temperatur bedingt ist, und führen somit zu einer UF-Fehlbilanz (Ultrafiltration = UF).

Ein anderer Problempunkt ist, dass es bei Änderungen der Standardeinstellungen, wie zum Beispiel des Volumenstroms der Dialysierflüssigkeit, auch als DF-Fluss bezeichnet, des blutseitigen Drucks, etc. oder bei dynamischen Druckänderungen, zum Beispiel infolge einer Alterung eines DF-Filters oder einer ähnlichen Einheit, trotz der eingesetzten Konstantdruckventile (Drosseln) zu Fehlbilanzen kommen kann. Grund ist, dass sich entsprechend dem jeweiligen Volumenstrom die Druckbedingungen im System ändern und es entsprechend zu Volumenänderungen kommen kann, die mittels der Bilanzierungseinrichtung nicht erfasst werden können.

Bisher wurde zur Kompensation von UF-Fehlmengen ein UF-Kompensationsfaktor in Form einer statischen Konstante in einer Steuerungssoftware hinterlegt. Dieser Kompensationsfaktor ist jedoch unabhängig von allen vorstehend genannten variablen Parametern wie Druck, Fluss und Temperatur, und stellt somit nur einen "mittleren Kompensationsfaktor" dar. Dieser ist je nach Einstellung der Maschine (Abweichungen von Standard-Dialyseeinstellungen und Standard-Umgebungsparametern) besser oder schlechter geeignet und ermöglicht eine exakte Kompensation von UF-Fehlmengen in der Regel nicht.

Zusammenfassend kann man sagen, dass es im Falle von Druck- und/oder Temperaturunterschieden in der Dialysierflüssigkeit, insbesondere vor und hinter der Bilanzkammer, zu Fehlbilanzierungen kommen kann. Druckunterschiede können aus verschiedenen Gründen resultieren, wie zum Beispiel Änderungen des DF-Flusses (Kalibrierung der Drosseln auf 500ml/min), Alterung von biologischen Komponenten (Abnutzung von DF-Filtern, Verblockung bzw. Aussetzung von Desinfektionsmitteln), Unterschieden hinsichtlich der Temperatur der Dialysierflüssigkeit relativ zur Umgebungstemperatur der Dialysemaschine, Änderungen des blutseitigen Druckes, etc. Solche Fehlbilanzierungen hängen demnach stark von an der Maschine eingestellten oder vorliegenden Parametern sowie von externen Einflussfaktoren ab und können Über- oder Unterfiltration des Patienten verursachen.

Ausgehend von dem vorstehend beschriebenen Stand der Technik liegt der vorliegenden Erfindung die Aufgabe zugrunde, die zuvor angeführten Nachteile zu beseitigen, insbesondere ein nicht beanspruchtes Verfahren und eine Dialysemaschine bereit zu stellen, mittels dem bzw. der derartige Fehlbilanzierungen verringert und bestenfalls vermieden werden können.

Die beanspruchte Erfindung wird durch die Merkmale des unabhängigen Anspruchs 1 definiert.

Nach der Erfindung wird diese Aufgabe vorzugsweise verfahrensseitig gelöst durch ein nicht beanspruchtes Verfahren zur Bestimmung einer Fehlbilanz, insbesondere einer Ultrafiltrations-Fehlbilanz, einer Dialysemaschine mit einem Dialysator, wobei zum Dialysator fließende Frischdialysierflüssigkeit und vom Dialysator abströmende Brauchdialysierflüssigkeit mittels zumindest einer Bilanzierungskammer bilanziert wird oder werden, wobei die Bilanzierungskammer ein mit Frischdialysierflüssigkeit beaufschlagtes frischstromseitiges Kammervolumen und ein mit Brauchdialysierflüssigkeit beaufschlagtes brauchstromseitiges Kammervolumen aufweist, welche mittels einer flexiblen Membran (hermetisch) voneinander getrennt sind und wechselseitig mit Dialysierflüssigkeit, also von einer Seite mit Frischdialysierflüssigkeit und von der anderen Seite mit Brauchdialysierflüssigkeit, beaufschlagt werden, wobei das Verfahren die Schritte aufweist:

- Bestimmen einer Druckdifferenz zwischen der frischstromseitigen Frischdialysierflüssigkeit und der brauchstromseitigen Brauchdialysierflüssigkeit,
- Ermitteln einer Bilanzabweichung anhand eines Vergleichs der bestimmten Druckdifferenz mit einer druckabhängigen Fehlbilanzrelation unter Berücksichtigung des eingestellten DF-Flusses,
- Bestimmung einer Korrekturrate anhand der ermittelten Bilanzabweichung und
- Ausgleich der Fehlbilanz durch Anwenden der bestimmten Korrekturrate.

Vorrichtungsseitig wird diese Aufgabe nach der Erfindung gelöst durch eine Dialysemaschine mit einem Dialysator und zumindest einer Bilanzierungskammer zur Bilanzierung von zum Dialysator fließender Frischdialysierflüssigkeit und vom Dialysator abströmender Brauchdialysierflüssigkeit, aufweisend ein erstes Druckerfassungsmittel (oder -einrichtung), das zum Erfassen eines auf der Frischstromseite der Bilanzkammer vorliegenden Drucks der Frischdialysierflüssigkeit angepasst ist, ein zweites Druckerfassungsmittel (oder -einrichtung), das zum Erfassen eines auf der Brauchstromseite der Bilanzkammer vorliegenden Drucks der Brauchdialysierflüssigkeit angepasst ist und eine Steuerungseinheit, die zum Vergleichen der erfassten Drücke unter Berücksichtigung des eingestellten DF-Flusses, Ermitteln einer Bilanzabweichung anhand eines Vergleichs der bestimmten Druckdifferenzen mit einer druckabhängigen Fehlbilanzrelation, Bestimmen einer Korrekturrate anhand der ermittelten Bilanzabweichung und Ausgleichen der Fehlbilanz durch Anwenden der bestimmten Korrekturrate angepasst ist.

Vereinfacht kann man sagen, dass die Grundidee, auf der die Erfindung beruht, ist, druckabhängige und flussabhängige - und bei besonderen Ausführungsformen zusätzlich oder alternativ temperaturabhängige - Fehlbilanzen zu kompensieren, indem die Druckverhältnisse vor und hinter der Bilanzkammer (man kann auch sagen die auf die Bilanzkammern jeweils wirkenden Drücke) bestimmt werden. Da eine Fehlbilanz eine druckabhängige Systemeigenschaft darstellt, kann diese durch Kenntnis des Druckunterschiedes kompensiert werden. Es ist ein besonderer Vorteil, dass so mit der Erfindung bei einer Dialysebehandlung entstehende Fehlbilanzen und insbesondere UF-Fehlbilanzen einfach, verlässlich und unaufwändig erfasst, bestimmt und ausgeglichen werden können. Dabei werden durch einen Regelalgorithmus, der mit den erfassten Druckwerten bzw. Druckdifferenzen und des eingestellten DF-Flusses, die störende Volumenunterschiede und damit Fehlbilanzen hervorrufen, als Eingangsvariablen arbeitet, eine Korrekturrate beispielsweise in Form eines Kompensationsflusses berechnet. Die Korrekturrate bzw. der Kompensationsfluss wird nach der Erfindung derart auf den Frischdialysierflüssigkeitsstrom und/oder den Brauchdialysierflüssigkeitsstrom angewendet, dass die vorliegende Fehlbilanz ausgeglichen wird.

Es ist ein besonderer Vorteil der Erfindung, dass in die Bestimmung der genannten Druckpotenziale außerdem Änderungen von Drücken aufgrund sich verändernder DF-Filter-Membranen (Dialysierflüssigkeit = DF), beispielsweise infolge von Alterung oder Verschleiß, sowie über die durch den blutseitigen Druck übertragene Druckkomponente auf die Dialysierflüssigkeitsseite eingehen. Da mit der Erfindung durch Erfassung und Analyse nur eines Parameters (oder bei anderen Ausführungsformen weniger Parameter) eine große Anzahl sich negativ auf die Bilanzierung auswirkender Einflüsse ausgeschaltet werden kann, ermöglicht die Erfindung hohe Robustheit und Wirtschaftlichkeit.

Vorteilhafte Ausführungsformen der Erfindung sind in den Unteransprüchen beansprucht und werden nachfolgend näher erläutert.

Bei einer nicht beanspruchten Ausführungsform des bevorzugten erfindungsgemäßen Verfahrens bewirkt in einem ersten Zyklus oder Betriebszeitraum in das frischstromseitige Kammervolumen einströmende Frischdialysierflüssigkeit eine Verformung der Membran. Dabei wird in dem brauchstromseitigen Kammervolumen befindliche Brauchdialysierflüssigkeit ausgetrieben. In einem zweiten Zyklus oder Betriebszeitraum bewirkt die in das brauchstromseitige Kammervolumen einströmende Brauchdialysierflüssigkeit eine Verformung der Membran und treibt die in dem frischstromseitigen Kammervolumen befindliche Frischdialysierflüssigkeit aus. Die beiden genannten Zyklen laufen während einer Dialysebehandlung fortwährend abwechselnd ab. Durch Vorsehen zweier Bilanzkammern, in denen die genannten Zyklen vertauscht sind, kann eine fortwährende Förderung und Bilanzierung von Dialysierflüssigkeit erzielt werden.

Vorzugsweise wird die Druckdifferenz zwischen der Frischdialysierflüssigkeit und der Brauchdialysierflüssigkeit bestimmt. Dies erfolgt vorzugsweise indem der Druck der Frischdialysierflüssigkeit und der Druck der Brauchdialysierflüssigkeit mittels Drucksensoren erfasst werden und die Differenz dieser erfassten Drücke bestimmt wird. In einer erfindungsgemäßen Dialysemaschine ist daher ein Drucksensor auf der Frischdialysierflüssigkeitsseite der Bilanzkammer eingebaut. Zusätzlich ist ein weiteres Druckerfassungsmittel auf der Brauchdialysierflüssigkeitsseite vorgesehen. Bei diesem handelt es sich vorzugsweise um einen weiteren zusätzlichen Drucksensor. Mit derartigen Druckerfassungsmitteln bzw. Sensoren ist eine Bestimmung der Druckverhältnisse und insbesondere von Druckunterschieden vor und hinter der Bilanzkammer, also auf der Frischseite und der Brauchseite, besonders einfach und effektiv möglich. Die Druckerfassung erfolgt nach der Erfindung möglichst in oder (unmittelbar) an der Bilanzkammer oder der Bilanzierungseinheit, derart, dass der dort wirkenden Druck möglichst unverfälscht aufgenommen wird.

Wie vorstehend dargelegt wurde, ist eine Fehlbilanz in erster Linie eine druckabhängige Systemeigenschaft. Zusätzlich ist jedoch die Kenntnis des Volumenstroms der Dialysierflüssigkeit, insbesondere die Kenntnis der Höhen der Frisch- und/oder Brauchdialysierflüssigkeitsströme, wichtig, da diese einen wesentlichen Einfluss auf das an den entsprechenden Strömungsabschnitten vorliegende Druckniveau haben.

Des Weiteren kann eine Kenntnis von in der Dialysierflüssigkeit vorliegenden Temperaturen und Temperaturverläufen von Vorteil sein. Bei einer Ausführungsform der Erfindung wird eine Flussbestimmung genutzt, um die Druckdifferenz zwischen der Frischdialysierflüssigkeit und der Brauchdialysierflüssigkeit zu bestimmen. Dabei wird bzw. werden ein Volumenstrom der Frischdialysierflüssigkeit und/oder ein Volumenstrom der Brauchdialysierflüssigkeit durch eine Drossel, vorzugsweise eine einstellbare Drossel, erfasst und mit einer volumenstromabhängigen Druckrelation für die Drossel verglichen. Bei einer anderen Ausführungsform, die alternativ oder zusätzlich zu der vorstehend beschriebenen Ausführungsform verwendet werden kann, kann eine Temperaturdifferenz zwischen der frischstromseitigen Frischdialysierflüssigkeit und der brauchstromseitigen Brauchdialysierflüssigkeit bestimmt werden. Es kann eine Bilanzabweichung anhand eines Vergleichs der bestimmten Temperaturdifferenz mit einer temperaturabhängigen Fehlbilanzrelation ermittelt werden, eine Korrekturrate anhand der ermittelten Bilanzabweichung bestimmt werden und eine Fehlbilanz durch Anwenden der bestimmten Korrekturrate ausgeglichen werden. Es können insbesondere Temperaturänderungen der Dialysierflüssigkeit vor und nach der Bilanzierungseinrichtung, welche in einer Volumendifferenz münden, mittels Temperatursensoren bestimmt werden. Zusammenfassend kann man auch sagen, dass bei einigen Ausführungsformen der Erfindung alternativ oder zusätzlich zu den genannten Druck- oder Druckdifferenzwerten Temperatur- und/oder Temperaturdifferenzwerte sowie der DF-Fluss, also der Volumenstrom der Frischdialysierflüssigkeit und/oder der Brauchdialysierflüssigkeit als Eingangsvariablen des Regelalgorithmus verwendet werden.

Zur Kompensation von zu viel oder zu wenig entnommenen Flüssigkeitsmengen durch die Bilanzierungseinheit kann eine Pumpe verwendet werden. Anders ausgedrückt wird eine Fehlbilanz ausgeglichen, indem mittels einer Pumpe ein Kompensationsvolumenstrom erzeugt wird, vorzugsweise indem der Kompensationsvolumenstrom mit einer Ultrafiltrationspumpe der Dialysemaschine erzeugt wird. Dabei ist es von besonderem Vorteil, dass eine solche in der Regel in der Dialysiermaschine schon vorhanden ist. Wird die Ultrafiltrationspumpe im Sinne der Erfindung zum Ausgleichen einer Fehlbilanz in der Bilanzkammer genutzt, fördert sie zu diesem Zweck nicht mehr eine vordefinierte Ultrafiltrationsrate, sondern eine um die Fehlbilanz der Bilanzkammer modifizierte Rate. Man kann dann auch sagen, dass die Ultrafiltrationspumpe einen um den Kompensationsvolumenstrom modifizierten Ultrafiltrationsvolumenstrom fördert.

Vorzugsweise wird bzw. werden die Druckdifferenz und/oder die Temperaturdifferenz zwischen dem Bilanzkammereingang auf der Brauchdialysierflüssigkeitsseite der Bilanzkammer und dem Bilanzkammerausgang auf der Frischdialysierflüssigkeitsseite der Bilanzkammer bestimmt.

Im Hinblick auf die Vorrichtung sieht die Erfindung bei einer Ausführungsform vor, dass das erste Druckerfassungsmittel ein Drucksensor oder eine vorzugsweise einstellbare Drossel ist und/oder das zweite Druckerfassungsmittel ein Drucksensor oder eine vorzugsweise einstellbare Drossel ist.

Bei der Dialysemaschine kann des Weiteren diese ein erstes Temperaturerfassungsmittel (oder -einrichtung), das zum Erfassen einer auf der Frischstromseite der Bilanzkammer vorliegenden Temperatur der Frischdialysierflüssigkeit angepasst ist, ein zweites Temperaturerfassungsmittel (odereinrichtung), das zum Erfassen einer auf der Brauchstromseite der Bilanzkammer vorliegenden Temperatur der Brauchdialysierflüssigkeit angepasst ist und eine Steuerungseinheit, die zum Vergleichen der erfassten Temperaturen, Ermitteln einer Bilanzabweichung anhand eines Vergleichs der bestimmten Temperaturdifferenzen mit einer temperaturabhängigen Fehlbilanzrelation, Bestimmen einer Korrekturrate anhand der ermittelten Bilanzabweichung und Ausgleichen der Fehlbilanz durch Anwenden der bestimmten Korrekturrate angepasst ist, aufweisen.

Mit der Dialysemaschine nach der Erfindung können in vorteilhafter Weise zahlreiche verschiedene Therapieformen, wie zum Beispiel Hämodialyse (HD), Hämofiltration (HF) oder Hämodiafiltration (HDF) durchgeführt werden, wobei Über- oder Unterultrafiltration des Patienten sicher vermieden werden kann.

Zusammenfassend kann festgehalten werden, dass Gegenstand der Erfindung unter anderem ein nicht beanspruchtes Verfahren zur Berechnung einer Fehlbilanz, insbesondere einer UF-Fehlbilanz, mittels eines Regelalgorithmus ist, der die Eingangsvariablen "Druck vor und nach der Bilanzkammer" sowie ggf. "Temperatur vor und nach der Bilanzkammer" und "DF-Fluss" verarbeitet und daraus einen Kompensationsfluss berechnet, der wiederum über die Ultrafiltrationspumpe umgesetzt wird. Mit der Erfindung werden unter anderem die folgenden Vorteile erzielt:

- Eine Bestimmung von durch die Bilanzierungseinheit hervorgerufenen und systembedingten Bilanzierungsabweichung ist möglich.
- Fehlbilanzen, insbesondere UF Fehlmengen, beispielsweise hervorgerufen durch das System der Bilanzierungskammer, können einfach und effektiv durch einen Regelalgorithmus kompensiert werden.
- Die für das erfindungsgemäße nicht beanspruchte Verfahren benötigten Eingangsparameter können besonders einfach durch an den meisten Dialysemaschinen teilweise schon vorhandene Sensoren oder mittels in einfacher Weise in eine Dialysemaschine zu integrierende Sensoren gewonnen werden.
- UF-Fehlmengen hervorgerufen durch einen Druckunterschied zwischen mehreren Bilanzierungskammern können durch den Algorithmus einfach berechnet und verringert oder verhindert werden.
- UF-Fehlmengen hervorgerufen durch einen Massenunterschied infolge eines Temperaturunterschieds zwischen Zu und Ablauf, d.h. zwischen der Frischseite und der Brauchseite, können durch den Algorithmus einfach berechnet und verringert oder verhindert werden.
- Ein Einfluss von von der Dialysierflüssigkeitstemperatur abweichenden Umgebungstemperaturen kann durch eine Auswertung von einfach in die Dialysemaschine zu integrierenden Temperatursensoren kompensiert werden.
- Eine Alterung von Dialysierflüssigkeitsfiltern kann durch Auswertung eines Drucksensors, insbesondere eines auf der Frischdialysierflüssigkeitsseite der Bilanzkammer angeordneten Drucksensors kompensiert werden.

Die Erfindung wird im Folgenden anhand beispielhafter, nicht einschränkender und in den angehängten Figuren gezeigter Ausführungsformen näher erläutert. Dabei zeigt:

Figur 1 eine schematische Darstellung eines Steueralgorithmus, der bei einem Verfahren und mit einer Dialysemaschine nach der Erfindung Verwendung findet,
Figur 2 ein schematisches Flussbild, das einen Teil einer erfindungsgemäßen Dialysemaschine zeigt,
Figur 3 eine schematische Darstellung einer Ausführungsform des Verfahrens wobei zwei Varianten gezeigt sind,
Figur 4 eine schematische Darstellung einer Verformung eines Bilanzkammerdeckels in einem Druck-Verformungs-Diagramm,
Figur 5 eine schematische Darstellung einer Ausdehnung von Silikonschläuchen einer Dialysemaschine in einem Druck-Verformungs-Diagramm,
Figur 6 eine schematische Darstellung der Temperaturabhängigkeit der Fehlbilanz in einem Volumen-Temperatur-Diagramm.

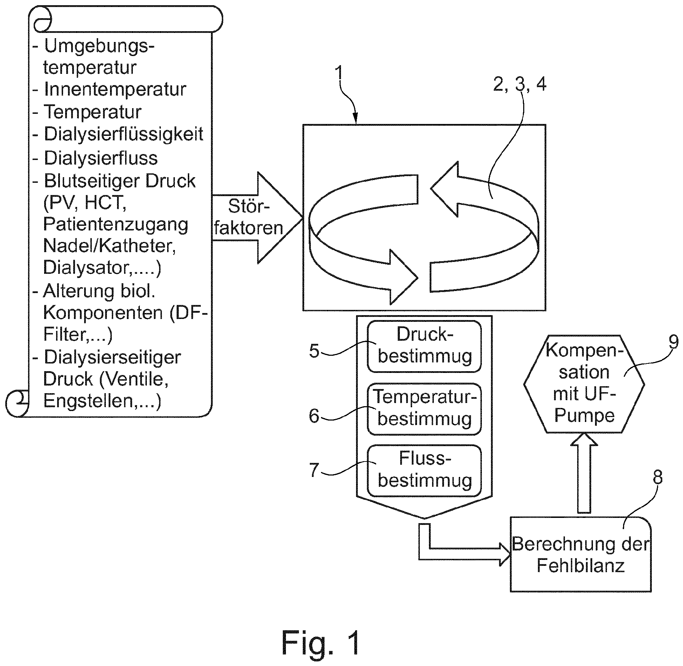

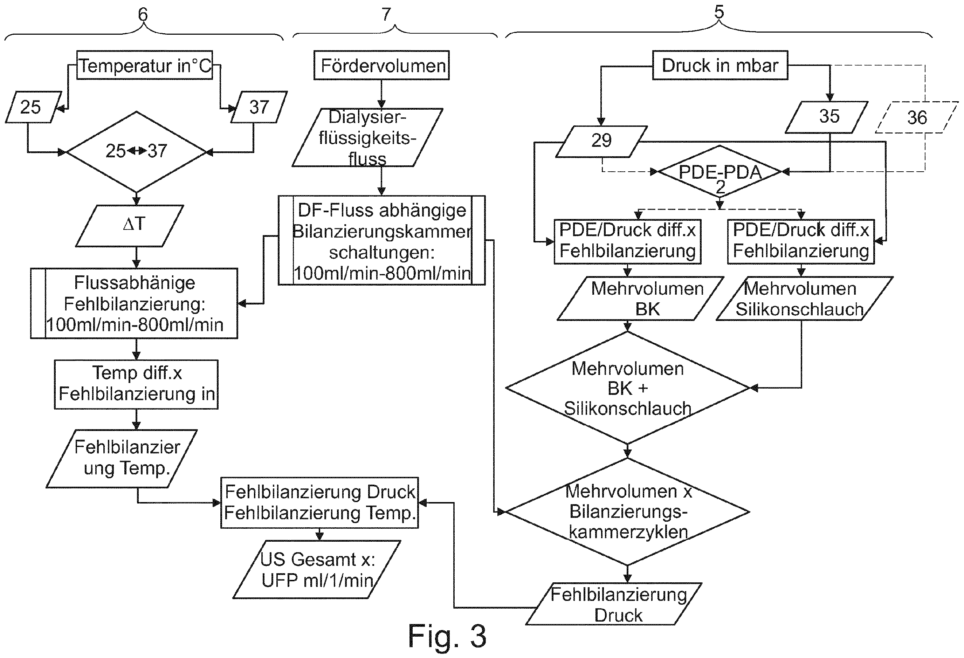

Figur 1 ermöglicht einen ersten Überblick über die Erfindung. Ein Dialysesystem, in diesem Fall eine Dialysemaschine 1 nach der Erfindung, weist eine Bilanzierungseinrichtung 2 auf, die ihrerseits wiederum zumindest eine Bilanzkammer 3, 4 umfasst. Die Bilanzierungseinrichtung 2 erstellt fortwährend eine Bilanzierung der die Dialysemaschine 1 durchlaufenden Dialyseflüssigkeit. In Figur 1 sind unterhalb der Dialysemaschine 1 die Erfassungen der eine Bilanzierung negativ beeinflussenden und eine Fehlbilanz hervorrufenden Faktoren aufgelistet, nämlich eine Druckbestimmung 5, eine Temperaturbestimmung 6 und eine Flussbestimmung 7 (siehe auch in Figur 3). Figur 1 verdeutlicht, wie anhand dieser Faktoren in einem Algorithmus 8 eine Fehlbilanz berechnet wird. Diese wird wiederum beim Erzeugen und Vorsehen einer Kompensation 9 zugrunde gelegt, die auf die Dialysemaschine 1 angewendet wird. Ganz links in Figur 1 sind Störfaktoren aufgelistet, die die Bilanzierung mittels der Bilanzkammer 3 verfälschen und eine Fehlbilanz hervorrufen. Diese Störgrößen sind unter anderem die Umgebungstemperatur, die Innentemperatur, die Temperatur der Dialyseflüssigkeit, der Fluss der Dialysierflüssigkeit, der blutseitige Druck (PV - venöser Druck, HCT, Patientenzugang Nadel/Katheter, Dialysator, etc.), die Alterung biologischer Komponenten, wie zum Beispiel der DF-Filter, etc. oder der dialysierseitige Druck beispielsweise an Ventilen oder Engstellen etc.

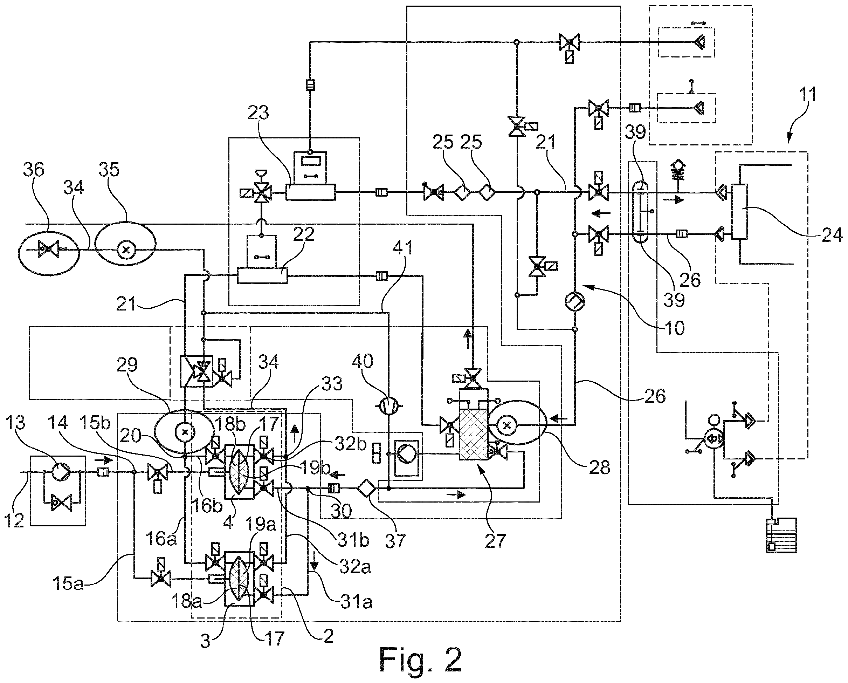

Figur 2 zeigt ein Flussschaltbild der Dialysemaschine 1. Angedeutet sind insbesondere deren Internfluidik 10 und eine Externfluidik 11 in Form einer extrakorporalen Blutleitung.

Die Internfluidik 10 besitzt einen Frischfluidanschluss 12, über den frische und unverbrauchte Dialysierflüssigkeit mittels einer Eingangsfluidpumpe 13 in das System geleitet wird. Der Frischfluidanschluss 12 verzweigt sich an einer Verzweigung 14 zu einer ersten Frischfluidzuleitung 15a und einer zweiten Frischfluidzuleitung 15b. Die erste Frischfluidzuleitung 15a führt zur ersten Bilanzkammer 3, während die zweite Frischfluidzuleitung 15b zur zweiten Bilanzkammer 4 führt.

Beide Bilanzkammern 3, 4 weisen jeweils eine Membran 17 auf, die die Bilanzkammer 3, 4 in eine Frischfluidkammer 18a,b und eine Brauchfluidkammer 19a,b unterteilt. Über die Frischfluidzuleitungen 15a,b in die Frischfluidkammern 18a,b einströmende Frischdialysierflüssigkeit verlässt die jeweilige Frischfluidkammer 18a,b über Frischfluidableitungen 16a,b. An einer Verzweigung 20 vereinigen sich die beiden Frischfluidableitungen 16a,b zu einer Frischfluidleitung 21. In dieser ist ein Drucksensor 29 angeordnet, mit dem der Druck in der Frischdialysierflüssigkeit ausgangsseitig der Bilanzierungseinrichtung 2 erfasst wird (Druck am Bilanzkammerausgang). Der Drucksensor 29 wird auch als Frischfluiddrucksensor bezeichnet.

Die Frischfluidleitung 21 führt über einen DF-Filter 22 und einen HDF-Filter 23 zu einem Dialysator 24. Zwischen diesem und dem HDF-Filter 23 ist ein Temperatursensor 25 eingebaut, mit dem die Temperatur der Frischdialysierflüssigkeit ausgangsseitig der Bilanziereinrichtung 2 erfasst werden kann. Im Dialysator 24 erfolgt in bekannter Weise eine Blutbehandlung oder Blutreinigung. Vom Dialysator 24 führt eine Brauchfluidleitung 26 zu einem Luftabscheider 27, in dem sich in der Dialysierflüssigkeit angesammelte Luft entfernt wird. Vor dem Luftabscheider 27 ist in der Brauchfluidleitung 26 ein Drucksensor 28 angeordnet, der den in der Brauchdialysierflüssigkeit vorliegenden Druck vor der Bilanziereinrichtung 2 erfasst. Nach dem Luftabscheider 27 ist in der Brauchfluidleitung 26 ein Temperatursensor 37 angeordnet, mit dem die Temperatur der Brauchdialysierflüssigkeit vor der Bilanzierungseinrichtung 2 erfasst werden kann.

Die Brauchfluidleitung 26 verzweigt sich an einer weiteren Verzweigung 30 zu einer ersten Brauchfluidzuleitung 31a und einer zweiten Brauchfluidzuleitung 31b. Die erste Brauchfluidzuleitung 31a führt zur ersten Bilanzkammer 3 in deren Brauchfluidkammer 19a, während die zweite Brauchfluidzuleitung 31b zur zweiten Bilanzkammer 4 in deren Brauchfluidkammer 19b führt. Über die Brauchfluidzuleitungen 31a,b in die Brauchfluidkammern 19a,b einströmende Brauchdialysierflüssigkeit verlässt die jeweilige Brauchfluidkammer 19a,b über Brauchfluidableitungen 32a,b. An einer Verzweigung 33 vereinigen sich die beiden Brauchfluidableitungen 32a,b zu einem Brauchfluidauslass 34. In diesem ist in einer ersten Ausführungsform ein weiterer Drucksensor 35, auch als Brauchfluiddrucksensor bezeichnet, angeordnet. Anstatt des Drucksensors 35 kann in einer zweiten Ausführungsform eine Drossel 36, auch als Konstantdruckventil bezeichnet, in den Brauchfluidauslass 24 eingesetzt sein. In Figur 3 ist die erste Variante mit durchgezogenen Linien dargestellt, die Drossel der zweiten Variante ist gestrichelt gezeigt.

Durch Vorsehen des Drucksensors 29 auf der Frischfluidseite der Bilanzeinrichtung direkt hinter deren Ausgang ist es möglich, den Druck an dieser Stelle kontinuierlich zu bestimmen. Dieser Druck ist direkt abhängig einerseits von der eingestellten Flussrate der Dialysierflüssigkeit im System und andererseits von alterungsbedingten Komponenten. Solche Komponenten können zum Beispiel die beiden Filter 22, 23 darstellen. Zusätzlich wirken sich Druckänderungen auf der Blutseite ebenfalls direkt auf den am Drucksensor 29 vorliegenden Druck aus. Alle Einflussfaktoren, die für eine unausgeglichene Bilanz sorgen, können somit als Druckänderung mit dem Drucksensor 29 gemessen werden. Zusätzlich kann der Drucksensor 28 herangezogen werden, um Druckänderungen vor allem der Blutseite zu erkennen. Allerdings sitzt dieser Sensor 28 bereits hinter dem Dialysator 24 (in Flussrichtung) und kann somit nicht zur Bestimmung einer Änderung des Druckabfalls z.B. über den DF-Filter 22 herangezogen werden. Da jedoch allgemein bekannt ist, dass nicht der absolute Druck am Bilanzkammereingang, sondern die Druckdifferenz zwischen Bilanzkammereingang und Bilanzkammerausgang zu einer Fehlbilanz führt, ist außerdem eine Bestimmung des Druckes am Bilanzkammerausgang erforderlich. Diese Druckbestimmung erfolgt nach dem vorliegenden Ausführungsbeispiel in einer ersten Form mittels des Drucksensors 35 oder in einer zweiten Ausführungsform mittels der Drossel 36. Vorteil der ersten Variante ist, dass der Druck mit dem Drucksensor 35 jederzeit messbar ist. Die zweite Variante mit der Drossel 36 ist zwar kostengünstiger, da diese bei bekannten Dialysemaschinen teilweise bereits im Flussschaltbild integriert ist, auf der anderen Seite ist der Druckabfall über die Drossel 36 flussabhängig. Zur Bestimmung der an der Bilanzierungseinrichtung anliegenden Druckdifferenz mittels der Drossel 36 muss daher zunächst deren flussabhängige Druckkennlinie aufgenommen werden oder bekannt sein. Bei einer auf einen konstanten Durchmesser eingestellte Drossel 36 ist somit der Druck hinter der Bilanzkammer 2 in Abhängigkeit des Flusses bekannt. Dies funktioniert jedoch nur für den Ausgang der Bilanzkammer 2, also die Brauchfluidseite, und nicht etwa auch für den Eingang der Bilanzkammer 2, also die Frischfluidseite, weil im Ausgang keine Komponenten vorhanden sind, die alterungsbedingten Druckänderungen unterliegen und keine weiteren Einflüsse von außen den Druck beeinflussen (wie beispielsweise der patientenindividuelle Druck der Blutseite).

Bei einer Ausführungsform der Erfindung wird zusätzlich die an der Bilanzierungseinrichtung 2 anliegende Temperaturdifferenz der Dialysierungsflüssigkeit, also die Temperaturdifferenz zwischen der Frischfluidseite und der Brauchfluidseite der Bilanzierungseinrichtung 2 bestimmt. Dies erfolgt mittels der Temperatursensoren 25 und 37. Man kann auch sagen, dass diese eine Differenz der Temperatur in der Dialysierflüssigkeit bestimmen, die beim Durchlaufen des Dialysators und der "Freistrecke" zwischen einem Spülbrückeneingang 38 und einem Spülbrückenausgang 39 verursacht wird. Da die Temperatur in einem Dialysezentrum üblicherweise wesentlich von der eingestellten DF-Temperatur abweicht, wird die Umgebungstemperatur in dem Dialysezentrum eine wesentliche Rolle bei dem Temperaturverlust der Dialysierflüssigkeit spielen. Eine solche Temperaturänderung wiederum führt unmittelbar zu einer Volumendifferenz bei der Bilanzierung.

Mit der Bestimmung der Druckdifferenz und der Temperaturdifferenz kann direkt auf die Fehlbilanz der Bilanzkammer geschlossen werden, da diese unmittelbar von Druck und Temperatur bzw. deren jeweiligen Differenzen abhängen. Durch Hinterlegen einer druck- und temperaturabhängigen Fehlbilanzkurve im System, also entsprechender Fehlbilanzrelationen, kann aus den ermittelten Druck- und Temperaturwerten bzw. -differenzen die tatsächliche Fehlbilanz der Bilanzkammer ermittelt werden.

Basierend auf der ermittelten Fehlbilanz wird nach der Erfindung im System eine Korrekturrate bestimmt. Um diese wird der Dialysierflüssigkeitsstrom im System modifiziert. Besonders vorteilhaft ist, wenn dazu eine im System vorhandene Ultrafiltrationspumpe 40 genutzt werden kann. Bei der in Figur 2 gezeigten Dialysemaschine 1 ist die Ultrafiltrationspumpe 40 in einer Ultrafiltrationsleitung 41 angeordnet, die den Brauchfluidauslass 34 mit der Brauchfluidleitung 26 zwischen dem Temperatursensor 37 und dem Luftabscheider 27 verbindet. Mit einem solchen System kann der Dialysierflüssigkeitsstrom im System modifiziert werden, indem die Ultrafiltrationsrate modifiziert wird. Dazu wird die Ultrafiltrationspumpe 40 genutzt.

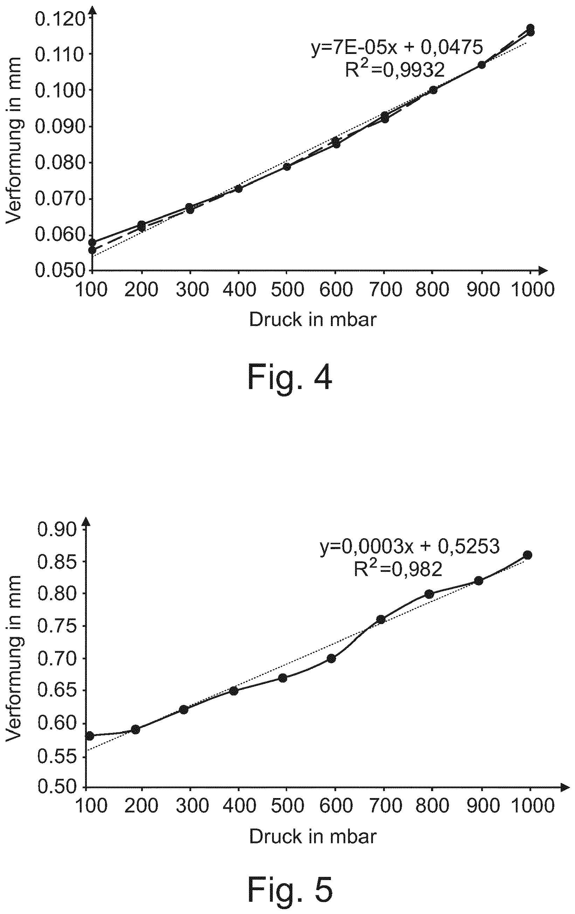

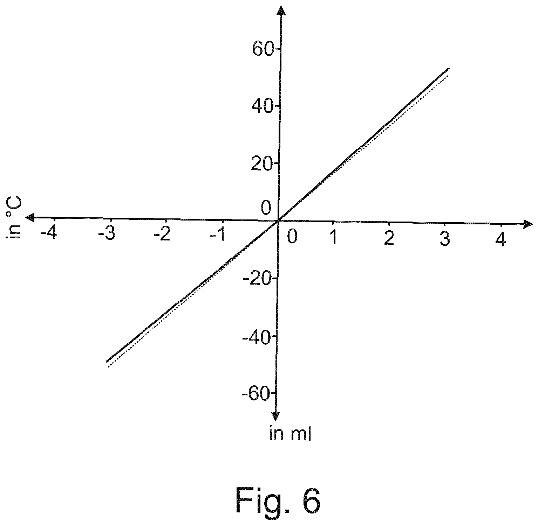

Steht eine Druckdifferenz an der Bilanzierungseinrichtung 2 fest, kann mittels der in den Figuren 4 und 5 dargestellten Relationen von Druck und Verformung (einmal der Membran 17 und einmal der Leitungen beispielsweise in Form von Silikonschläuchen) die zur Ausgleich erforderliche Flussrate bestimmt werden. Grundlage dazu ist eine Vermessung der Bauteile in verschiedenen Druckbereichen. Dies ist in den Berechnungen hergeleitet, die in den Diagrammen der Figuren 4 und 5 vermerkt sind. Ähnliches gilt, wenn eine an der Bilanzierungseinrichtung 2 anliegende Temperaturdifferenz ermittelt wurde. Dann kann die in Figur 6 gezeigte Relation zwischen Temperaturdifferenz und Fehlbilanz zu deren Ausgleich genutzt werden. Die dem Diagramm der Figur 6 zugrundeliegenden Formel und beispielhaften Konstanten sind im Folgenden gezeigt:

$$D = (a_0 + a_1 T + a_2 T^2 + a_3 T^3 + a T^4 + a_5 T^5) / (1 + bT) ,$$

mit

b = 0,016887230, a0 = 999,83952,
a1 = 16,952577, a2 = -0,0079905127,
a3 = -0,000046241757, a4 = 0,0000000105846 und
a5 = -0,000000000281, alle Konstanten für 0-150°C

Bezugszeichen

| | |
|---|---|
| 1 | Dialysiermaschine |
| 2 | Bilanzierungseinrichtung |
| 3 | erste Bilanzkammer |
| 4 | zweite Bilanzkammer |
| 5 | Druckbestimmung |
| 6 | Temperaturbestimmung |
| 7 | Flussbestimmung |
| 8 | Algorithmus |
| 9 | Kompensation |
| 10 | Internfluidik |
| 11 | Externfluidik |
| 12 | Frischfluidanschluss |
| 13 | Eingangsfluidpumpe |
| 14 | Verzweigung |
| 15a,b | Frischfluidzuleitung |

| | |
|---|---|
| 16a,b | Frischfluidableitung |
| 17 | Membran |
| 18a,b | Frischfluidkammer |
| 19a,b | Brauchfluidkammer |
| 20 | Verzweigung |
| 21 | Frischfluidleitung |
| 22 | DF-Filter |
| 23 | HDF-Filter |
| 24 | Dialysator |
| 25 | Temperatursensor |
| 26 | Brauchfluidleitung |
| 27 | Luftabscheider |
| 28 | Drucksensor |
| 29 | Drucksensor/Frischfluiddrucksensor |
| 30 | Verzweigung |
| 31a,b | Brauchfluidzuleitung |
| 32a,b | Brauchfluidableitungen |
| 33 | Verzweigung |
| 34 | Brauchfluidauslass |
| 35 | Drucksensor/ Brauchfluiddrucksensor |
| 36 | Drossel/Konstandruckventil |
| 37 | Temperatursensor |
| 38 | Spülbrückeneingang |
| 39 | Spülbrückenausgang |
| 40 | Ultrafiltrationspumpe |
| 41 | Ultrafiltrationsleitung |

**Patentansprüche**

1. Dialysemaschine (1) mit einem Dialysator (24) und zumindest einer Bilanzierungskammer (3, 4) zur Bilanzierung von zum Dialysator (24) fließender Frischdialysierflüssigkeit und vom Dialysator (24) abströmender Brauchdialysierflüssigkeit, aufweisend ein erstes Druckerfassungsmittel, das zum Erfassen eines auf der Frischstromseite der Bilanzkammer vorliegenden Drucks der Frischdialysierflüssigkeit angepasst ist, **gekennzeichnet durch** ein zweites Druckerfassungsmittel, das zum Erfassen eines auf der Brauchstromseite der Bilanzkammer vorliegenden Drucks der Brauchdialysierflüssigkeit angepasst ist und eine Steuerungseinheit, die zum Vergleichen der erfassten Drücke zwischen Frischdialysierflüssigkeit und Brauchdialysierflüssigkeit, Ermitteln einer Bilanzabweichung anhand eines Vergleichs der bestimmten Druckdifferenzen mit einer druckabhängigen Fehlbilanzrelation, Bestimmen einer Korrekturrate anhand der ermittelten Bilanzabweichung und Ausgleichen der Fehlbilanz durch Anwenden der bestimmten Korrekturrate angepasst ist.

2. Dialysemaschine nach Anspruch 1, **dadurch gekennzeichnet, dass** das erste Druckerfassungsmittel ein Drucksensor (29) oder eine vorzugsweise einstellbare Drossel ist und/oder das zweite Druckerfassungsmittel ein Drucksensor (28) oder eine vorzugsweise einstellbare Drossel ist.

3. Dialysemaschine nach Anspruch 1 oder 2, **dadurch gekennzeichnet, dass** diese ein erstes Temperaturerfassungsmittel, das zum Erfassen einer auf der Frischstromseite der Bilanzkammer (3, 4) vorliegenden Temperatur der Frischdialysierflüssigkeit angepasst ist, ein zweites Temperaturerfassungsmittel, das zum Erfassen einer auf der Brauchstromseite der Bilanzkammer (3, 4) vorliegenden Temperatur der Brauchdialysierflüssigkeit angepasst ist und eine Steuerungseinheit aufweist, die zum Vergleichen der erfassten Temperaturen, Ermitteln einer Bilanzabweichung anhand eines Vergleichs der bestimmten Temperaturdifferenzen mit einer temperaturabhängigen Fehlbilanzrelation, Bestimmen einer Korrekturrate anhand der ermittelten Bilanzabweichung und Ausgleichen der Fehlbilanz durch Anwenden der bestimmten Korrekturrate angepasst ist.

**Claims**

1. Dialysis machine (1) with a dialyser (24) and at least one balancing chamber (3, 4) for balancing fresh dialysis fluid flowing to the dialyser (24) and used dialysis fluid flowing out of the dialyser (24), having a first pressure detection means, which is suitable for detecting a pressure of the fresh dialysis fluid present on the fresh flow side of the balance chamber, **characterised by** a second pressure detection means, that is suitable for detecting a pressure of the used dialysis fluid present on the used flow side of the balance chamber and a control unit which is suitable for comparing the detected pressures between fresh dialysis fluid and used dialysis fluid, ascertaining a balance deviation based on a comparison of the determined pressure differences with a pressure-dependent imbalance relationship, determining a correction rate based on the ascertained balance deviation and compensating for the imbalance by applying the determined correction rate.

2. Dialysis machine according to claim 1, **characterised in that** the first pressure detection means is a pressure sensor (29) or a preferably adjustable throttle and/or the second pressure detection means as a pressure sensor (28) or a preferably adjustable throttle.

3. Dialysis machine according to claim 1 or 2, **characterised in that** it has a first temperature detection means that is suitable for detecting a temperature of the fresh dialysis fluid present on the fresh flow side of the balance chamber (3, 4), a second temperature detection means that is suitable for detecting a temperature of the used dialysis fluid present on the used flow side the balance chamber (3, 4) and a control unit that is suitable for comparing the detected temperatures, ascertaining a balance deviation based on a comparison of the determined temperature differences with a temperature-dependent imbalance relationship, determining a correction rate based on the determined balance deviation and compensating the imbalance by applying the determined correction rate.

**Revendications**

1. Machine de dialyse (1) avec un dialyseur (24) et au moins une chambre de bilan (3, 4) pour effectuer le bilan du liquide de dialyse frais s'écoulant jusqu'au dialyseur (24) et du liquide de dialyse usé sortant du dialyseur (24), présentant un premier moyen de détection de pression, qui est adapté pour détecter une pression du liquide de dialyse frais présente sur le côté d'écoulement frais de la chambre de bilan, **caractérisée par** un second moyen de détection de pression, qui est adapté pour détecter une pression du liquide de dialyse usé présente sur le côté d'écoulement usé de la chambre de bilan et une unité de commande, qui est adaptée pour comparer les pressions détectées entre le liquide de dialyse frais et le liquide de dialyse usé, déterminer un écart de bilan à l'aide d'une comparaison des différences de pression déterminées avec une relation de bilan erroné dépendant de la pression, déterminer un taux de correction à l'aide de l'écart de bilan déterminé et compenser le bilan erroné en appliquant le taux de correction déterminé.

2. Machine de dialyse selon la revendication 1, **caractérisée en ce que** le premier moyen de détection de pression est un capteur de pression (29) ou de préférence un étranglement réglable et/ou **en ce que** le second moyen de détection de pression est un capteur de pression (28) et/ou de préférence un étranglement réglable.

3. Machine de dialyse selon la revendication 1 ou 2, **caractérisée en ce qu'**elle présente un premier moyen de détection de température, qui est adapté pour détecter une température du liquide de dialyse frais présente sur le côté d'écoulement frais de la chambre de bilan (3,4), un second moyen de détection de température, qui est adapté pour détecter une température du liquide de dialyse usé présente sur le côté d'écoulement usé de la chambre de bilan (3, 4) et une unité de commande, qui est adaptée pour comparer les températures détectées, déterminer un écart de bilan à l'aide d'une comparaison des différences de température déterminées avec une relation de bilan erroné dépendant de la température, déterminer un taux de correction à l'aide de l'écart de bilan déterminé et compenser le bilan erroné en appliquant le taux de correction déterminé.

- Umgebungstemperatur
- Innentemperatur
- Temperatur
- Dialysierflüssigkeit
- Dialysierfluss
- Blutseitiger Druck
  (PV, HCT,
  Patientenzugang
  Nadel/Katheter,
  Dialysator,....)
- Alterung biol.
  Komponenten (DF-
  Filter,...)
- Dialysierseitiger
  Druck (Ventile,
  Engstellen,...)

Störfaktoren

1

2, 3, 4

Druck-
bestimmug

5

Temperaturbestimmug

6

Flussbestimmug

7

Kompensation
mit UF-
Pumpe

9

Berechnung der
Fehlbilanz

8

**Fig. 1**

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20050000868 A1